# EUROPEAN PATENT APPLICATION

(11) **EP 4 763 096 A1**
(43) Date of publication of application: **24.06.2026**
(21) Application number: 24383427.2
(22) Date of filing: 20.12.2024
(51) Int. Cl.: A61B 10/00

(54) **INGESTIBLE DEVICE FOR TAKING A SAMPLE OF A DIGESTIVE SYSTEM**

(71) Applicant: Fundación Tecnalia Research & Innovation, 20009 Donastia-San Sebastían Gipuzkoa (ES)
(72) Inventor: GARCIA LIZARRIBAR, Andrea Alicia, 20009 DONOSTIA - SAN SEBASTIAN - GIPUZKOA (ES); MONTEJO ESTEVEZ, Manuel, 20009 DONOSTIA - SAN SEBASTIAN - GIPUZKOA (ES); OLALDE GRAELLS, Beatriz, 20009 DONOSTIA - SAN SEBASTIAN - GIPUZKOA (ES)
(74) Representative: Balder IP Law, S.L.

(57) **Abstract**

The invention relates to an ingestible device (100) for taking a sample of a target location of a digestive system (200), the target location having pH within a first pH range and pressure within a first pressure range; the device comprising an elastic cover (102) enclosing an inner space (101), the elastic cover comprising an opening (106) for entrance of a sample into the inner space; the device comprising a first additional cover (103) degradable at the first pH range; the first additional cover (103) covering the elastic cover and compressing the elastic cover by applying a pressure on the elastic cover greater than a greatest pressure of the first pressure range so that, upon degradation of the first additional cover (103) in the target location of a digestive system, volume of the inner space (101) increases for forcing a sample to enter the inner space (101) through the opening.

## Description

### TECHNICAL FIELD

The present invention is encompassed within the field of ingestible devices for taking samples of digestive systems.

### BACKGROUND

Obtaining samples of the digestive system, for example, of the small intestine allows performing accurate diagnosis and effective management of various diseases (infections, Crohn's disease, celiac disease, cancer, polyps, etc.). Invasive techniques such as endoscopy and enteroscopy allow direct visualization as well as obtaining samples of intestinal tissue. Less invasive solutions for obtaining samples, for example, ingestible pills, are currently being investigated and are desirable for taking samples from the digestive system.

### SUMMARY

An aspect of the disclosure relates to an ingestible device for taking a sample of a target location of a digestive system (e.g., of a gastrointestinal tract), the target location having pH within a first pH range and a pressure within a first pressure range; the device comprising an elastic cover enclosing an inner space, the elastic cover comprising an opening for entrance of a sample into the inner space; the device comprising a first additional cover degradable at the first pH range; the first additional cover covering the elastic cover and compressing the elastic cover by applying a pressure on the elastic cover greater than a greatest pressure of the first pressure range so that, upon degradation of the first additional cover in the target location of a digestive system, volume of the inner space increases for forcing a sample to enter the inner space through the opening.

The ingestible device (e.g., an ingestible pill) is a device to be ingested by an organism, which has a digestive system, as if the device were food, so that the ingestible device goes along the same path of a digestive system as food. In some embodiments, the ingestible device is to be ingested by a human. In some embodiments, the ingestible device is to be ingested by an animal having a digestive system, e.g., a dog, a cow, a horse, a sheep or a goat.

The ingestible device is configured to take a sample of a target location of a digestive system having pH within a first pH range and a pressure within a first pressure range. The target location of the digestive system is, for example, a stomach, a small intestine or a large intestine. For example, the target location is a duodenum, an ileum, a cecum, a colon or a rectum.

The fact that the pH is within a first pH range does not necessarily mean that the pH is not the same in all places of the target location, although in some embodiments, the pH is not the same in all places of the target location. Similarly, the fact that the pressure of the target location is within a first pressure range does not necessarily mean that the pressure is not the same in all places of the target location, although in some embodiments, the pressure is not the same in all places of the target location.

The fact that the pH is within a first pH range does not necessarily mean that the pH is different at different times, although in some embodiments, the pH is different at different times. Similarly, the fact that the pressure of the target location is within a first pressure range does not necessarily mean that the pressure is different at different times, although in some embodiments, the pressure is different at different times.

Since the cover enclosing the inner space is elastic, the elastic cover can be compressed to decrease the volume of the inner space. In particular, the ingestible device comprises a first additional cover which exerts a compression force on the elastic cover.

The first additional cover is degraded at the first pH range, and hence is degraded in the target location of the digestive system. Therefore, when the first additional cover is in the target location, the first additional cover is degraded, so that the compression force applied by the first additional cover to the elastic cover disappears.

In addition, because the pressure in the target location of the digestive system is smaller than the compression pressure applied by the first additional cover to the elastic cover, the elastic cover expands towards an original shape of the elastic cover thus increasing volume of the inner space and generating a suction force in the opening of the elastic cover to suction, into the inner space, a sample from the target location of the digestive system. The suction increases the speed of sample taking, thereby taking a sample from a smaller portion of the digestive than if said suction force were not applied. In addition, the suction allows taking more complete samples because the suction forces certain samples into the inner space of the device that, if the suction were not applied, would not enter the inner space.

In some embodiments, the target location is a location of a small intestine, for example, a location of jejeum, ileum or cecum.

pH of the small intestine, in general, is of at least four and at most 8.5.

In some embodiments, the ingestible device comprises a second additional cover degradable at a second pH range of a digestive system, the second pH range being different than the first pH range, wherein the second pH range comprises at least a portion greater than a greatest value of the first pH range and/or at least a portion smaller than a smallest value of the first pH range (e.g., the first pH range and the second pH range do not overlap each other); the second additional cover covering the first additional cover.

A second additional cover which covers the first additional cover allows protecting the first cover from degrading in a location of the digestive system which has pH in the first pH range but which is not the target location, so that the first additional cover is not degraded in an undesired location of the digestive system and the ingestible device does not take a sample in said undesirable location.

In particular, the second additional cover is degraded in a location of the digestive system previous to the target location of the digestive system. In the present disclosure, a location of the digestive system is considered to be previous to another location of the digestive system if the location is configured to process ingested food before the another location. More specifically, the esophagus is previous to the stomach, the stomach is previous to the duodenum, the duodenum is previous to the jejenum, the jejenum is previous to the ileum, the ileum is previous to the cecum, the cecum is previous to the colon and the colon is previous to the rectum. In the present disclosure, a location of the digestive system is considered to be posterior to another location of the digestive system if the location is configured to process food after the another location.

For example, if the target location is the small intestine, and the esophagus has a pH range similar to that of the target location, a second additional cover may protect the first additional cover while the ingestible device is in the esophagus, and the second additional cover may be degraded at a location posterior to the esophagus and previous to the target location, e.g., the stomach.

Therefore, the second additional cover is stable (in the sense that the second additional cover does not stop protecting the first additional cover from premature degradation due to pH) in a location which has similar pH as the target location.

The fact that the first pH range and the second pH range do not overlap each other, means that the first pH range and the second pH range do not share any pH value.

In some embodiments, a smallest value of the first pH range is greater than a greatest value of the second pH range (e.g., the smallest value of the first pH range is six; e.g., the greatest value of the second pH range is three). These embodiments, are advantageous in those cases in which the digestive system has a location, previous to the target location, having pH within the first pH range, and the digestive system comprises a location between said locations which has a different, e.g., smaller pH. For example, in the case of a digestive system of a human, pH of the saliva in the mouth/esophagus may be equal to pH in a jejenum location. If the target location is the jejenum, the ingestible device may comprise a second additional cover to protect the first additional cover from degradation in the mouth/esophagus. More particularly, the second additional cover is degraded at pH smaller to that of the target location, for example, at pH reached in the stomach or in the duodenum, e.g., pH smaller than six, four or three.

The second additional cover remains stable (in the sense that the second additional cover does not suffer degradation to such an extent that the second additional cover stops protecting the first additional cover from degradation due to pH) while the ingestible device is in the location with the first pH range where the sample is not intended to be taken, so that the first additional cover is adequately protected from premature degradation by pH.

The first additional cover remains stable (in the sense that the first additional cover does not suffer degradation to such an extent that the compression force exerted by the first additional cover to the elastic cover is decreased and/or that enables that a sample enters the inner space through the opening of the elastic cover) while the ingestible device is in the location with the second pH range, so that sample is not undesirably taken in the location having the second pH range.

In some embodiments, the ingestible device comprises a third additional cover degradable at a third pH range of a digestive system, the third pH range being different than the second pH range, wherein the third pH range comprises at least a portion greater than a greatest value of the second pH range and/or at least a portion smaller than a smallest value of the second pH range; the third additional cover covering the second additional cover.

A third additional cover which covers the second additional cover allows protecting the second cover from degrading in a location of the digestive system which has pH in the second pH range but which is not the location where it is intended that the second additional cover is degraded, so that the second additional cover is not degraded in an undesired location of the digestive system.

The third additional cover remains stable (in the sense that the third additional cover does not suffer degradation to such an extent that the third additional cover stops protecting the second additional cover from degradation due to pH) while the ingestible device is in the location with the second pH where the second additional cover is not intended to be degraded, so that the second additional cover is adequately protected from premature degradation by pH.

In some embodiments, the target location of the digestive system (i.e., the location of the digestive system from which a sample is intended to be obtained) is the ileum of a person, more specifically a location of the ileum which is after a maximum pH of the small intestine. Before reaching the ileum, the ingestible device goes through other two locations of the digestive system which have similar pH to that of the target location, namely a location of the mouth/esophagus with saliva and a location of the jejenum and/or duodenum.

The third additional cover may be degradable at pH of a location of the digestive system located between the mouth/esophagus and the jejenum/duodenum where the first pH range is present. It is known that pH at locations posterior to the mouth/esophagus and previous to the jejenum/duodenum is smaller than that of the target location. Therefore, the third additional cover can be degraded at pH smaller than that of the target location.

In some embodiments, the third additional cover is degradable at pH smaller than four, preferably at pH smaller than three.

The third additional cover remains stable at the second pH range (in the sense that the third additional cover does not suffer degradation to such an extent that the third additional cover stops protecting the second additional cover from degradation due to pH), so that the second additional cover is adequately protected, by the third additional cover.

The second additional cover remains stable at the third pH range (in the sense that the second additional cover does not suffer degradation to such an extent that the second additional cover stops protecting the first additional cover from degradation due to pH), so that the second additional cover is not degraded in the location where the third additional cover is degraded.

In some embodiments, the smallest value of the second pH range is greater than the greatest value of the third pH range. After the third additional cover is degraded, the ingestible device enters a location of the digestive system where the pH is greater than the smallest value of the second pH range, so that the second additional cover is degraded.

In some embodiments, the second additional cover is degradable at pH greater than six.

To prevent/minimize degradation of the first additional cover before reaching the target location, the second additional cover may be configured accordingly (e.g., cover material, cover porosity, cover thickness, etc.).

In particular, because pH of locations of the digestive system can be estimated in advance, it can be estimated how quickly a determined cover will be degraded. Therefore, the configuration of the cover may be estimated/determined so that the cover is degraded when the ingestible device reaches a determined location of the digestive system.

Undesirable degradation of the first additional cover may be minimized/prevented if the first additional cover remains stable (in the sense that the first additional cover does not suffer degradation to such an extent that the compression force exerted by the first additional cover to the elastic cover is decreased and/or that enables that a sample enters the inner space through the opening of the elastic cover) above a certain pH which is smaller than the maximum pH of the small intestine.

In some embodiments, the first additional cover is degradable at pH smaller than seven.

After the second additional cover is degraded, the ingestible device enters the target location, where the first additional cover is degraded. After degradation of the first additional cover, a sample enters the inner space of the ingestible device by suction.

In some embodiments, the target location of the digestive system (i.e., the location of the digestive system from which a sample is intended to be obtained) is the cecum of a person. Before reaching the cecum, the ingestible device goes through other two locations of the digestive system which have pH similar to that of the target location, namely a location of the mouth/esophagus with saliva and a location of the jejenum and/or duodenum.

In those embodiments in which the target location is the cecum, the third additional cover may be the same as that described for the embodiments in which the target location is the ileum.

The second additional cover is configured to degrade before reaching the target location, and to be stable (in the sense that the second additional cover does not stop protecting the first additional cover from premature degradation due to pH) at the third pH range. For example, the smallest value of the second pH range is greater than the greatest value of the third pH range, and the second additional cover is degradable at pH greater than the smallest value of the second pH range. After the third additional cover is degraded, the ingestible device enters a location of the digestive system where the pH is greater than the smallest value of the second pH range, so that the second additional cover is degraded.

In some embodiments, the second additional cover is degradable at pH greater than seven.

In some embodiments, the first additional cover is stable (in the sense that the first additional cover does not suffer degradation to such an extent that the compression force exerted by the first additional cover to the elastic cover is decreased and/or that enables that a sample enters the inner space through the opening of the elastic cover) at pH greater than a smallest value of the second pH range. Thereby, the first additional cover is not degraded in the location of the digestive system where the second additional cover is degraded.

In some embodiments, the first additional cover is degradable at pH smaller than six.

In some embodiments, the first additional cover covers the opening for blocking access of a sample to the opening.

For example, the first additional cover may compress a complete contour of the opening so that a watertight (e.g., an airtight) coupling between the elastic cover and the first additional cover completely surrounds the opening thus preventing liquid (and gas if the coupling is airtight) from reaching the opening.

The blocking of the access of the sample may be achieved by other means, for example, by a first additional cover which completely covers the elastic cover.

In some embodiments, the second additional cover completely covers the first additional cover. In this way, the second additional cover protects the whole first additional cover from undesired degradation by pH.

In some embodiments, the third additional cover completely covers the second additional cover. In this way, the third additional cover protects the whole second additional cover from undesired degradation by pH.

In some embodiments, the ingestible device comprises a check valve in the opening. The check valve is configured to allow that sample (e.g., liquid) enters the inner space of the device and to block exit of sample material from the inner space.

In some embodiments, the ingestible device comprises a gelling component in the inner space, the gelling component being for gelling a liquid sample. A gelling component configured for converting a liquid sample into gel is advantageous for further blocking exit of sample material from the inner space.

In some embodiments, the opening of the elastic cover is surrounded by a ridge in an outer side of the elastic cover, the ridge comprising lateral holes connecting the opening with an exterior of the elastic cover. Said ridge allows separating the opening from walls of the digestive system (e.g., intestine walls) so that the walls do not block access of sample to the opening. Preferably, the ridge is more rigid than the elastic cover.

In some embodiments, the ingestible device comprises an absorbing component in the inner space, the absorbing component being for absorbing a liquid sample. Thereby, retention of the liquid sample in the inner space of the ingestible device is enhanced.

### BRIEF DESCRIPTION OF THE DRAWINGS

To complete the description and to provide for a better understanding of the disclosure, a set of drawings is provided. Said drawings form an integral part of the description and illustrate embodiments of the disclosure, which should not be interpreted as restricting the scope of the disclosure, but just as examples of how the disclosure can be carried out. The drawings comprise the following figures:
Figure 1 shows an ingestible device in accordance with embodiments.
Figure 2 shows an inner structure of an ingestible device in accordance with embodiments.
Figure 3 shows a digestive system in accordance with embodiments.
Figure 4 shows a graph representing pH evolution along a path of an ingestible device in a digestive system in accordance with embodiments and locations of degradation of additional covers of an ingestible device in accordance with embodiments.
Figure 5 shows a graph representing pH evolution along a path of an ingestible device in a digestive system in accordance with embodiments and locations of degradation of additional covers of an ingestible device in accordance with embodiments.
Figure 6 shows a graph representing pH evolution along a path of an ingestible device in a digestive system in accordance with embodiments and locations of degradation of additional covers of an ingestible device in accordance with embodiments.
Figure 7 shows enlargement of an inner space of an elastic cover of an ingestible device in accordance with embodiments.
Figure 8 shows an inner space and an elastic cover of an ingestible device in accordance with embodiments.
Figure 9 shows an elastic cover provided with a ridge surrounding an opening of the elastic cover in accordance with embodiments.

### DETAILED DESCRIPTION

The following description is not to be taken in a limiting sense but is given solely for the purpose of describing the broad principles of the invention. Embodiments of the invention will be described by way of example, with reference to the above-mentioned drawings.

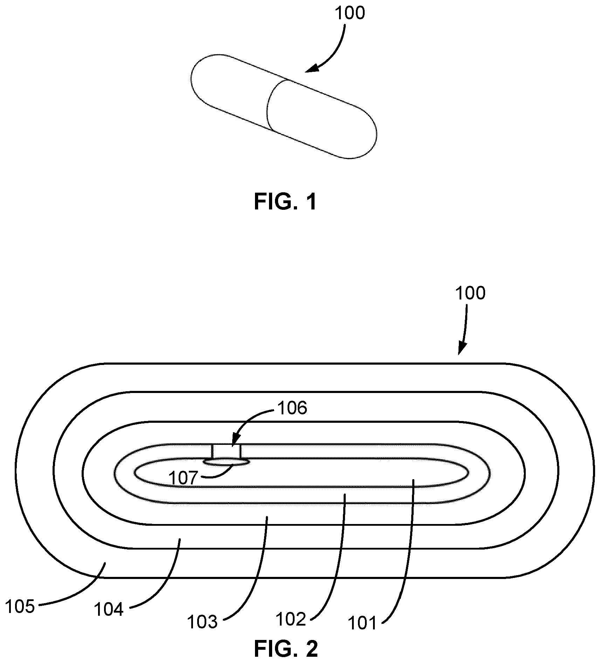

Figure 1 shows an ingestible device 100 in accordance with embodiments. The ingestible device 100 is ingestible, for example, by a human.

As shown in Figure 2, the ingestible device 100 comprises an elastic cover 102, a first additional cover 103, a second additional cover 104 and a third additional cover 105. Although, as explained below, in some embodiments the third additional cover 105 is omitted.

The elastic cover 102 completely encloses an inner space 101 except for an opening 106. The opening 106 connects the inner space 101 with the outside of the elastic cover 102. A lid 107 regulates entrance of sample into the inner space 101.

The first additional cover 103 covers the elastic cover 102, for example, completely covers the elastic cover 102.

In some alternative embodiments (not shown), the first additional cover 103 covers just some portions of the elastic cover 102. For example, the first additional cover 103 is ring-shaped. For example, the ring-shaped first additional cover covers the opening 106

The second additional cover 104 covers the first additional cover 103, for example, completely covers the first additional cover 103.

The third additional cover 105 covers the second additional cover 104, for example, completely covers the second additional cover 104.

The elastic cover 102 is made of a material ingestible by an animal (e.g., by a human) and which recovers an original shape upon releasing a compression force applied by the first additional cover so that suction forces a sample to enter the inner space through the opening. For example, the elastic cover 102 is made of thermoplastic polyurethane and/or of low density polyethylene.

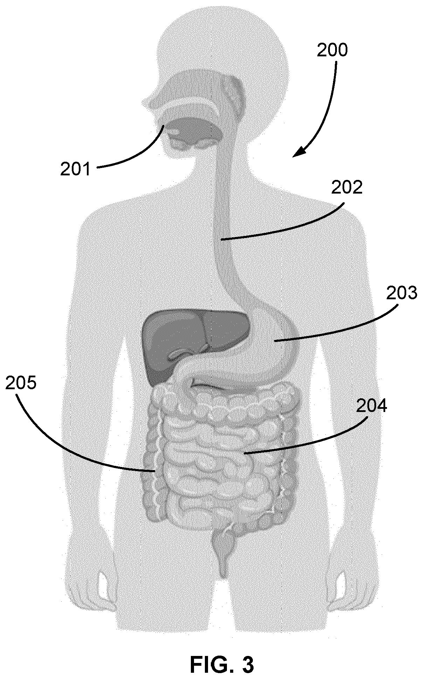

Figure 3 shows a digestive system 200 of a human. Upon ingestion of the ingestible device 100, the ingestible device 100 enters the mouth 201, then the esophagus 202, then the stomach 203, then the small intestine 204 and then the large intestine 205.

In general, the mouth 201 has pH of at least 6.5 and at most 7.5, the stomach has pH of at least 1 and at most 2.5, the duodenum has pH of at least 5.6 and at most 6.6, the jejenum has pH of at least 6.6 and at most 7.6, the ileum pH of at least 7 and at most 8, the cecum has pH of at least 5.5 and at most 6.5, the colon has pH of at least 6.37 and at most 7.04, and the rectum has pH of at least 7 and at most 7.3.

By estimating pH ranges of locations of the digestive system and a time that the ingestible device will be in each of the locations, it can be determined the features of each additional cover of the ingestible device (e.g., material, thickness, porosity, pore diameter, fiber diameter and crystallinity of each cover) to control the locations where each of the additional cover(s) degrade.

A pH range is defined by two endpoints, i.e., a smallest number and a greatest number.

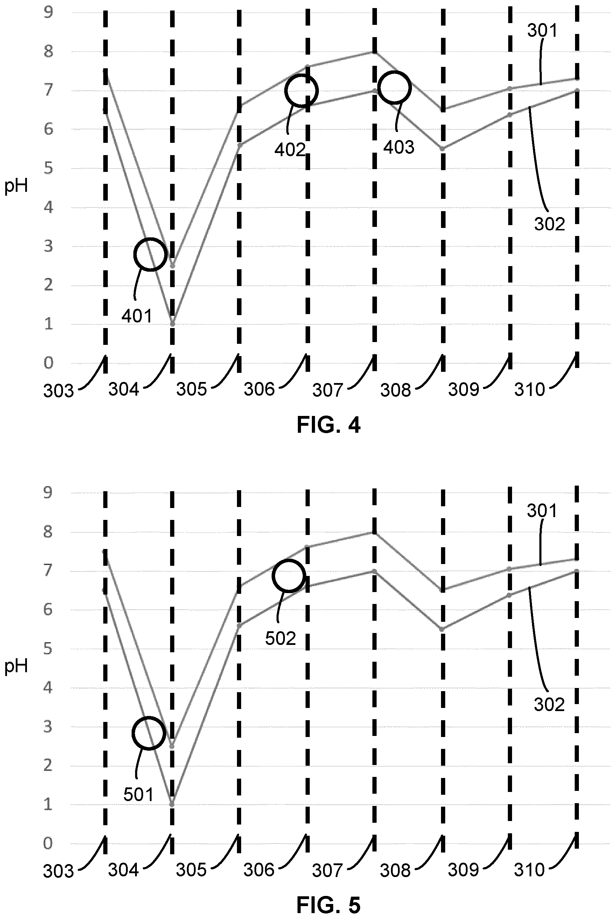

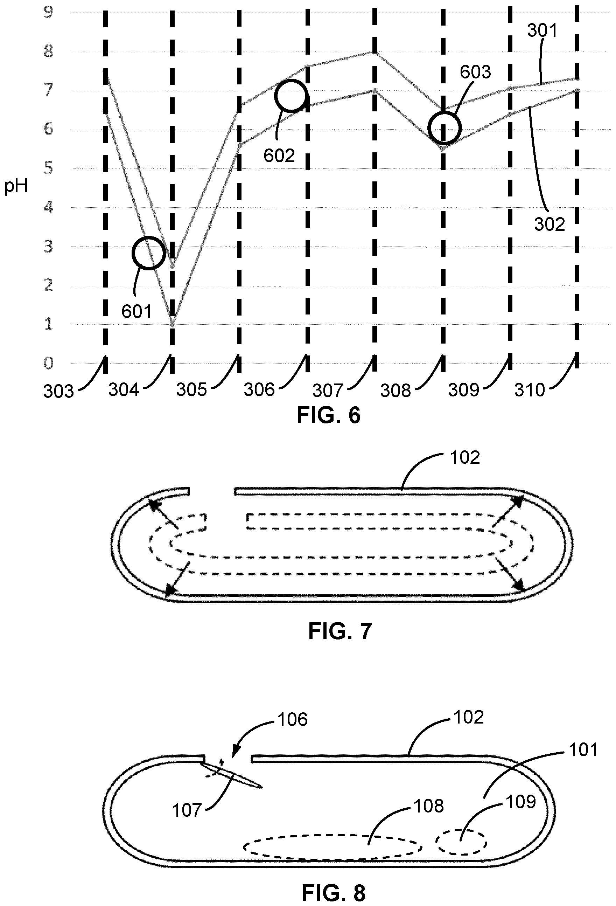

Figures 4, 5 and 6 show greatest pH 301 and smallest pH 302 in different locations of a digestive system 200. Specifically, Figures 4, 5 and 6 show:
- smallest pH and greatest pH of saliva 303 in mouth and/or esophagus,
- smallest pH and greatest pH in a stomach location 304,
- smallest pH and greatest pH in a duodenum location 305,
- smallest pH and greatest pH in a jejenum location 306,
- smallest pH and greatest pH in an ileum location 307,
- smallest pH and greatest pH in a cecum location 308,
- smallest pH and greatest pH in a colon location 309, and
- smallest pH and greatest pH in a rectum location 309.
Each of Figures 4, 5 and 6 refers to a different ingestible device 100. The ingestible device referred to in Figures 4 and 6 comprise three additional covers, whereas the ingestible device referred to in Figure 5 has just two additional covers.

Figure 4 shows three locations 401, 402, 403 of the digestive system where different additional covers of the ingestible device 100 are degraded. In Figure 4, the target location from which a sample is to be taken is 403, which is a location of the ileum.

Figure 4 shows a location 401 of the stomach where a third additional cover 105 of the ingestible device is degraded. The third additional cover 105 remains stable (in the sense that the third additional cover does not stop protecting the second additional cover from premature degradation due to pH) until reaching location 401 of the stomach. At pH of location 401, the third additional cover 105 is degraded so that the second additional cover 104 is exposed to the pH of the digestive system 200. The material of the third additional cover 105 may be any material ingestible by an animal (e.g., by a human) and which is degraded at sufficient speed when exposed to a stomach pH, for example, at pH of between 1.5 and four, for example, at pH of between 1.5 and three. For example, the material of the third additional cover 105 is collagen and/or chitosan.

The second additional cover 104 of the ingestible device 100 referred to in Figure 4 remains stable (in the sense that the second additional cover does not stop protecting the first additional cover from premature degradation due to pH) until reaching a duodenum and/or jejenum location 402 in which the second additional cover 104 is degraded. At pH of the location 402, the second additional cover 104 is degraded so that the first additional cover 103 is exposed to the pH of the digestive system 200. The material of the second additional cover 104 may be any material ingestible by an animal (e.g., a human) and which is degraded at sufficient speed when exposed to pH in the duodenum and/or jejenum, for example, at pH greater than five, for example, at pH greater than six. For example, the material of the second additional cover 104 is at least one of: polylactic acid, polyglycolic acid, copolymer of polylactic acid and copolymer of polyglycolic acid.

The first additional cover 103 of the ingestible device 100 referred to in Figure 4 remains stable (in the sense that the first additional cover does not suffer degradation to such an extent that the compression force exerted by the first additional cover to the elastic cover is decreased and/or that enables that a sample enters the inner space through the opening of the elastic cover) until reaching an ileum location 403 where the first additional cover 103 is degraded. The stability may be achieved by degradation of the second additional cover 104 at a location having a greater pH to that of location 403 (e.g., a pH greater than 7). At the pH of the location 403, the first additional cover 103 is degraded so that the compression exerted by the first additional cover 103 on elastic cover 103 disappears and samples can enter the inner space 101 through the opening 106 of the elastic over 102 by means of suction. The material of the first additional cover 103 may be any material ingestible by an animal (e.g., a human) and which is degraded at sufficient speed when exposed to pH in the ileum, for example, at pH smaller than seven. For example, the material of the first additional cover 103 is at least one of: gelatin, alginic acid and hypromellose.

Figure 5 shows two locations 501, 502 of the digestive system where different additional covers of the ingestible device 100 are degraded. In Figure 5, the target location from which a sample is to be taken is 502, which is a location of the jejenum.

The second additional cover 104 of the ingestible device referred to in Figure 5 can be made of the same material as the third additional cover 105 of the ingestible device referred to in Figure 4 because both covers remain stable until reaching a location 401, 501 of the stomach.

The first additional cover 103 of the ingestible device 100 referred to in Figure 5 remains stable (in the sense that the first additional cover does not suffer degradation to such an extent that the compression force exerted by the first additional cover to the elastic cover is decreased and/or that enables that a sample enters the inner space through the opening of the elastic cover) until reaching a jejenum location 502. At pH of the location 502, the first additional cover 103 is degraded so that the compression exerted by the first additional cover 103 on elastic cover 102 disappears and samples can enter the inner space 101 through the opening 106 of the elastic over 102. The material of the first additional cover 103 may be any material ingestible by an animal (e.g., a human) and which is degraded at sufficient speed when exposed to pH in the jejenum, for example, at pH greater than six. For example, the material of the first additional cover 103 is at least one of: polylactic acid, polyglycolic acid, copolymer of polylactic acid and copolymer of polyglycolic acid.

Figure 6 shows three locations 601, 602, 603 of the digestive system where different additional covers of the ingestible device 100 are degraded. In Figure 6, the target location from which a sample is to be taken is 603, which is a location of the cecum.

The third additional cover 105 of the ingestible device referred to in Figure 6 can be made of the same material as the third additional cover 105 of the ingestible device referred to in Figure 4 because both covers remain stable until reaching a location 401, 601 of the stomach.

The second additional cover 104 of the ingestible device referred to in Figure 6 can be made of the same material as the second additional cover 104 of the ingestible device referred to in Figure 4 because both covers remain stable until reaching a location 402, 602 of the duodenum/jejenum. The second additional cover 104 of the ingestible device referred to in Figure 6 is, for example, degradable at pH greater than seven.

The first additional cover 103 of the ingestible device 100 referred to in Figure 6 remains stable (in the sense that the first additional cover does not suffer degradation to such an extent that the compression force exerted by the first additional cover to the elastic cover is decreased and/or that enables that a sample enters the inner space through the opening of the elastic cover) until reaching a cecum location 603. At the pH of the location 603, the first additional cover 103 is degraded so that the compression exerted by the first additional cover 103 on elastic cover 102 disappears and samples can enter the inner space 101 through the opening 106 of the elastic over 102. The material of the first additional cover 103 may be any material ingestible by an animal (e.g., a human) and which is degraded at sufficient speed when exposed to pH in the cecum, for example, at pH smaller than six.

Upon degradation of the first additional cover 103, a compression force exerted by the first additional cover 103 to the elastic cover 102 disappears. Since pressure at the location of the digestive system where the first additional cover 103 is degraded applies a compression force to the elastic cover 102 smaller than the compression force applied by the first additional cover 103, the elastic cover 102 expands towards an original shape of the elastic cover 102 (e.g., a shape of the elastic cover 102 at 1 atm), so that volume of the inner space 101 of the elastic cover 102 increases thus generating a suction which forces a sample to go through the opening 106 and enter the inner space 101. Figure 7 (in which lid 107 is not shown) illustrates the expansion of the elastic cover 102.

Pressure in a location of a digestive system between esophagus and rectum may be slightly greater than atmospheric pressure, e.g., of at least 0.1 kPa and at most 6 kPa greater than atmospheric pressure (e.g., 1 atm), for example, of at least 0.3 kPa and at most 6 kPa greater than atmospheric pressure. For example, pressure in the small intestine is of at least 0.5 kPa and at most 3 kPa greater than atmospheric pressure, for example, of at least 1 kPa and at most 2 kPa greater than atmospheric pressure.

Figure 8 shows greater detail of the elastic cover 102 and of its inner space 101. Figure 8 shows a moveable (e.g., pivotable) lid 107 which can be part of a check valve. The moveable lid may be configured to close the opening when the inner space 101 contains sufficient amount of sample. For example, when the sample volume within the inner space 101 is big enough to contact the lid 107, the sample within the inner space 101 may apply force to the lid 107 to move the lid 7 from an open position (which allows that sample enters the inner space) to a closed position (in which the lid blocks the opening 106, so that further sample cannot enter the inner space 101).

In addition, upon degradation of the first additional cover 103, the first additional cover 103 may stop blocking access of liquid to the inner space 101, so that sample liquid can start entering the inner space 101.

A gelling component 108 may be in the inner space 101. When a liquid sample and the gelling component are in contact, the liquid sample is gelled. The resulting gel applies a force to the lid 107 greater than the force applied by the liquid sample to the lid 107, thus allowing enhancing liquid-tightness of the closure of the opening 106. Some examples of the material of the gelling component are one or more of: alginate, gelatin, chitosan and collagen.

An absorbing component 109 may be in the inner space 101. When a liquid and the absorbing component are in contact, the liquid sample is absorbed by the absorbing component 109, thus enhancing retention of the sample in the inner space 101.

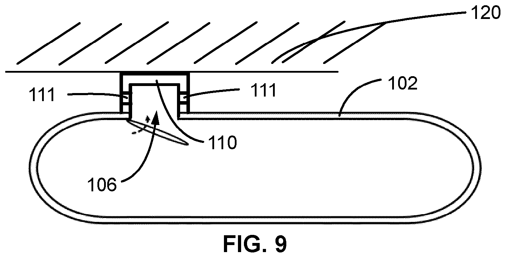

Figure 9 shows a ridge 110 surrounding the opening 106 in an outer side of the elastic cover 102. The ridge 110 is in contact with an intestine wall 120. Specifically, a part of the ridge located opposite the opening 106 is in contact with the intestine wall 120. The ridge 110 comprises lateral holes 111 (i.e., holes between the opening and the part of the ridge 110 opposite the opening 106) which connect the opening 106 with the exterior of the elastic cover 102. Thereby, sample material can go through the lateral holes 111 when the ridge 110 is in contact with the intestine wall 120. The ridge 110 is more rigid than the elastic cover 102, so that greater force is required to deform the ridge 110 and hence the resistance to blocking the opening 106 by deformation of the ridge 110 is increased.

In this text, the term "comprises" and its derivations (such as "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. An ingestible device (100) for taking a sample of a target location of a digestive system (200), the target location having pH within a first pH range and pressure within a first pressure range; the device (100) comprising an elastic cover (102) enclosing an inner space (101), the elastic cover (102) comprising an opening (106) for entrance of a sample into the inner space (101); the device (100) comprising a first additional cover (103) degradable at the first pH range; the first additional cover (103) covering the elastic cover (102) and compressing the elastic cover (102) by applying a pressure on the elastic cover (102) greater than a greatest pressure of the first pressure range so that, upon degradation of the first additional cover (103) in the target location of a digestive system (200), volume of the inner space (101) increases for forcing a sample to enter the inner space (101) through the opening (106).

2. The ingestible device (100) of claim 1, wherein the target location is a location of a small intestine (204).

3. The ingestible device (100) of any one of the previous claims, comprising a second additional cover (104) degradable at a second pH range of a digestive system (200), the second pH range being different than the first pH range, wherein the second pH range comprises at least a portion greater than a greatest value of the first pH range and/or at least a portion smaller than a smallest value of the first pH range; the second additional cover (104) covering the first additional cover (103).

4. The ingestible device (100) of claim 3, wherein a smallest value of the first pH range is greater than a greatest value of the second pH range.

5. The ingestible device (100) of claim 3, comprising a third additional cover (105) degradable at a third pH range of a digestive system (200), the third pH range being different than the second pH range, wherein the third pH range comprises at least a portion greater than a greatest value of the second pH range and/or at least a portion smaller than a smallest value of the second pH range; the third additional cover (105) covering the second additional cover (104).

6. The ingestible device (100) of claim 5, wherein the third additional cover (105) is degradable at pH smaller than four, preferably at pH smaller than three.

7. The ingestible device (100) of claim 5 or 6, wherein the second additional cover (104) is degradable at pH greater than six.

8. The ingestible device (100) of any one of claims 5 to 7, wherein the first additional cover (103) is degradable at pH smaller than seven.

9. The ingestible device (100) of claim 5 or 6, wherein the second additional cover (104) is degradable at pH greater than seven.

10. The ingestible device (100) of claim 5, 6 or 9, wherein the first additional cover (103) is degradable at pH smaller than six.

11. The ingestible device (100) of any one of the previous claims, wherein the first additional cover (103) covers the opening (106) for blocking access of a sample to the opening.

12. The ingestible device (100) of any one of the previous claims, wherein the first additional cover (103) completely covers the elastic cover (102).

13. The ingestible device (100) of any one of the previous claims, wherein the second additional cover (104) completely covers the first additional (103) cover and/or the third additional cover (105) completely covers the second additional cover (104).

14. The ingestible device (100) of any one of the previous claims, wherein the ingestible device comprises a check valve in the opening (106).

15. The ingestible device (100) of any one of the previous claims, the ingestible device comprising a gelling component (108) in the inner space (101), the gelling component (108) being for gelling a liquid sample.
